# EUROPEAN PATENT SPECIFICATION

(11) **EP 3 833 189 B1**
(45) Date of publication and mention of the grant of the patent: **13.09.2023**
(21) Application number: 19758908.8
(22) Date of filing: 08.08.2019
(51) Int. Cl.: C05F 11/08, A01N 63/22, C05F 17/20, C12N 1/20, C12R 1/10

(54) **A BACTERIAL COMPOSITION CAPABLE OF DEGRADING COMPLEX CARBOHYDRATES**
ZUM ABBAU VON KOMPLEXEN KOHLENHYDRATEN FÄHIGE BAKTERIELLE ZUSAMMENSETZUNG
COMPOSITION BACTÉRIENNE CAPABLE DE DÉGRADER DES GLUCIDES COMPLEXES

(30) Priority: 08.08.2018 EP 18188108
(43) Date of publication of application: 16.06.2021
(73) Proprietor: Monaghan Mushrooms Ireland Unlimited Company, County Monaghan H18 FW95 (IE)
(72) Inventor: WILSON, Jude, Monaghan, H18 FW95 (IE); UCCELLO, Andrea, Monaghan, H18 FW95 (IE)
(74) Representative: Murgitroyd & Company
(86) International application number: PCT/EP2019/071373
(87) International publication number: WO 2020/030763

(56) References cited:
- EP-A1- 0 537 418
- KIYOHIKO NAKASAKI ET AL: "A Newly Isolated Thermophilic Bacterium, Bacillus Licheniformis HA1 to Accelerate the Organic Matter Decomposition in High Rate Composting", COMPOST SCIENCE AND UTILIZATION, vol. 2, no. 2, 1 March 1994 (1994-03-01), pages 88-96, XP055514665, US ISSN: 1065-657X, DOI: 10.1080/1065657X.1994.10771142
- KIYOHIKO NAKASAKI ET AL: "The Use of Bacillus Licheniformis HA1 To Accelerate Composting of Organic Wastes", COMPOST SCIENCE AND UTILIZATION, vol. 4, no. 4, 1 September 1996 (1996-09-01), pages 47-51, XP055514667, US ISSN: 1065-657X, DOI: 10.1080/1065657X.1996.10701852
- REY MICHAEL W ET AL: "Complete genome sequence of the industrial bacterium Bacillus licheniformis and comparisons with closely related Bacillus species", GENOME BIOLOGY, BIOMED CENTRAL LTD., LONDON, GB, vol. 5, no. 10, 13 September 2004 (2004-09-13), pages r77-1, XP021012839, ISSN: 1465-6906, DOI: 10.1186/GB-2004-5-10-R77
- EMEKA A. OKOROMA ET AL: "Identification and characterisation of a Bacillus licheniformis strain with profound keratinase activity for degradation of melanised feather", INTERNATIONAL BIODETERIORATION & BIODEGRADATION, vol. 74, 13 August 2012 (2012-08-13), pages 54-60, XP055514673, AMSTERDAM, NL ISSN: 0964-8305, DOI: 10.1016/j.ibiod.2012.07.013
- JURADO MACARENA ET AL: "Exploiting composting biodiversity: Study of the persistent and biotechnologically relevant microorganisms from lignocellulose-based composting", BIORESOURCE TECHNOLOGY, vol. 162, 4 April 2014 (2014-04-04), pages 283-293, XP028654796, ISSN: 0960-8524, DOI: 10.1016/J.BIORTECH.2014.03.145
- JIAQI XU ET AL: "Inoculation with Compost-Born Thermophilic Complex Microbial Consortium Induced Organic Matters Degradation While Reduced Nitrogen Loss During Co-Composting of Dairy Manure and Sugarcane Leaves", WASTE AND BIOMASS VALORIZATION, 11 April 2018 (2018-04-11), XP055514814, NL ISSN: 1877-2641, DOI: 10.1007/s12649-018-0293-y
- SIVAKUMAR NALLUSAMY ET AL: "Isolation and characterization of cellulolytic Bacillus licheniformis from compost", AFRICAN JOURNAL OF BIOTECHNOLOGY, vol. 15, no. 43, 26 October 2016 (2016-10-26), pages 2434-2446, XP055514670, DOI: 10.5897/AJB2016.15641
- K George Abraham: "Extraction of Lignin Peroxidase Enzyme from Bacteria Isolated from the Mangrove Wood", PARIPEX -INDIAN JOURNAL OF RESEARCH, 1 June 2016 (2016-06-01), pages 7-9, XP055514738, India Retrieved from the Internet: URL:https://www.worldwidejournals.com/pari pex/recent_issues_pdf/2016/June/June_2016_ 1465913839__116.pdf [retrieved on 2018-10-12]
- DATABASE WPI Week 200210 Thomson Scientific, London, GB; AN 2002-069736 XP002785631, -& JP 2001 261474 A (AMANO CORP) 26 September 2001 (2001-09-26)
- DATABASE WPI Week 201171 Thomson Scientific, London, GB; AN 2011-M47734 XP002785632, -& CN 102 174 423 A (INST AGRIC RESOURCES & REGIONAL PLANNING) 7 September 2011 (2011-09-07)
- DATABASE WPI Week 201520 Thomson Scientific, London, GB; AN 2015-139607 XP002785633, -& CN 104 261 912 A (SHANXI KINGSHINE FERTILIZER CO LTD) 7 January 2015 (2015-01-07)
- DATABASE WPI Week 201533 Thomson Scientific, London, GB; AN 2015-22906E XP002785634, -& CN 104 357 357 A (HEILONGJIANG ACAD SCI INST MICROBIOLOGY) 18 February 2015 (2015-02-18)
- DATABASE WPI Week 201626 Thomson Scientific, London, GB; AN 2016-18853G XP002785635, -& JP 2016 039786 A (UNIV ISHIKAWA PREFECTURAL) 24 March 2016 (2016-03-24)
- DATABASE WPI Week 201707 Thomson Scientific, London, GB; AN 2016-71314X XP002785636, -& CN 106 083 228 A (CHINA NAT RICE RES INST) 9 November 2016 (2016-11-09)
- DATABASE WPI Week 201760 Thomson Scientific, London, GB; AN 2017-50637S XP002785637, -& CN 106 957 807 A (UNIV GUANGXI) 18 July 2017 (2017-07-18)
- DATABASE WPI Week 201821 Thomson Scientific, London, GB; AN 2018-11475L XP002785638, -& CN 107 653 200 A (UNIV HUAZHONG AGRIC) 2 February 2018 (2018-02-02)

## Description

### Field of the invention

The present invention relates to a bacterial composition, and can be used to augment compost.

### Background of the invention

CN 102 174 423 A describes the bacterial strain *Bacillus licheniformis* CH15. The strain is gram- and catalase-positive, degrades cellulose, hemicellulose and lignin when used for preparing a soil fertiliser composition by composting straw. CN 106 957 807 A describes *Bacillus licheniformis* TA65 for the inoculation of compost.

K. Nakasaki et al. (Compost Science and Utilization 1996,4, 47-51) disclose the use of *Bacillus licheniformis* HA1 for composting.

Compost for the cultivation of *Agaricus bisporus* is formulated from wheat straw, poultry manure, horse manure, gypsum and ammonium sulphate. The raw materials undergo a three phase composting process to prepare a substrate that is selective for the cultivation of *A. bisporus.* Phase 1 involves the fermentation of the raw materials. This is characterised by the biochemical breakdown of the complex organic matter by microorganisms, which causes changes in the compost chemistry. At this stage, high temperatures (≥75°C) are reached due to the high microbial activity. Phase 2 involves the pasteurisation of the Phase 1 compost to destroy any potential human pathogens and the removal of ammonia, which is detrimental to mushroom growth, from the compost (to less than 5 ppm). Phase 3 involves the inoculation of the Phase 2 compost with grain spawn (which contains *A. bisporus* spores) and the colonisation of the compost by the *A. bisporus* mycelium.

Mushroom compost contains a broad composition of complex carbohydrates, such as lignin, hemicellulose and cellulose, which are continually degraded throughout the cropping cycle. The degradation of these complex polymers into utilisable sugar monomers requires a variety of enzymes. These carbohydrate hydrolysis enzyme activities in compost vary throughout the production process. However, little is currently known about the particular micro-organisms that mediate these enzymatic processes.

Several authors have demonstrated that bacteria that are commonly present in mushroom compost exhibit enzymatic activities in the breakdown of complex carbon sources such as lignin, cellulose and hemicellulose. Moreover, it has been suggested that some fungi may also promote mushroom growth through the digestion of these complex carbohydrates and the subsequent release of CO₂, which has been correlated with mushroom biomass increase.

Several attempts have been made to improve the content of simple sugars that are easily utilised by *Agaricus bisporus* mycelium in the mushroom compost. Both fungi and bacteria have been used, with some encouraging results regarding improvements to the total composting time and final mushroom yield. Such studies were carried out at a small scale, under controlled conditions, and focused their attention only on thermophilic microorganisms. No data are available about the success of these microorganisms to augment compost at commercial scale.

### Summary of the invention

In this specification, it will be understood that the terms 'comprise' 'comprises' or 'comprising' may be replaced by the terms 'consists of', 'consisting of', 'consists essentially of' or consisting essentially of'.

In this specification, it will be understood, in a general context, that complex carbohydrates are polysaccharides, ideally containing alpha or beta type glycosidic bonds, and have longer chains compared to simple carbohydrates.

It will be appreciated that the scope is in accordance with the claims. Accordingly, the present invention is concerned with a bacterial composition comprising at least one strain of *Bacillus licheniformis* comprising the *Bacillus licheniformis* strain deposited at NCIMB Ltd. with accession number NCIMB 42690 and/or the *Bacillus licheniformis* strain deposited at NCIMB Ltd. with accession number NCIMB 42691 as defined in claim 1. Further features are provided in accordance with the dependant claims.

The bacterial composition is capable of degrading complex carbohydrates selected from the group comprising: hemicellulose, cellulose, and combinations thereof.The bacterial composition is further capable of degrading lignin protein.The bacterial composition is further capable of assimilating ammonia.

The at least one strain of *Bacillus licheniformis* is gram positive, catalase positive, and oxidase positive.

The at least one strain of *Bacillus licheniformis* is rod shaped. Optionally, in particular the cells of the at least one strain of *Bacillus licheniformis* are rod shaped. Further optionally, the cells of the at least one strain of *Bacillus licheniformis* have the appearance of short bacilli, with filaments.

Accordingly, the at least one strain of *Bacillus licheniformis* comprises a gram positive, catalase positive and oxidase positive rod shaped strain.

Optionally, the at least one strain of *Bacillus licheniformis* comprises a gram positive, catalase positive and oxidase positive strain, the cells of which have the appearance of short bacilli, with filaments.

Additionally, the at least one strain of *Bacillus licheniformis* has a white coloured , rough surfaced colony morphology.

Optionally, the at least one strain of *Bacillus licheniformis* has an irregular form colony morphology. Further optionally, the at least one strain of *Bacillus licheniformis* has a flat elevation colony morphology. Further optionally, the at least one strain of *Bacillus licheniformis* has a curled margin colony morphology.

Optionally, the at least one strain of *Bacillus licheniformis* comprises a gram positive, catalase positive and oxidase positive rod shaped strain that has an irregular form colony morphology. Further optionally, the at least one strain of *Bacillus licheniformis* comprises a gram positive, catalase positive and oxidase positive rod shaped strain that has a white coloured, rough surfaced, irregular form colony morphology.

Optionally, the at least one strain of *Bacillus licheniformis* comprises a gram positive, catalase positive and oxidase positive rod shaped strain that has a flat elevation colony morphology. Further optionally, the at least one strain of *Bacillus licheniformis* comprises a gram positive, catalase positive and oxidase positive rod shaped strain that has a white coloured, rough surfaced, flat elevation colony morphology.

Optionally, the at least one strain of *Bacillus licheniformis* comprises a gram positive, catalase positive and oxidase positive rod shaped strain that has a curled margin colony morphology. Further optionally, the at least one strain of *Bacillus licheniformis* comprises a gram positive, catalase positive and oxidase positive rod shaped strain that has a white coloured, rough surfaced, curled margin colony morphology.

Optionally, the at least one strain of *Bacillus licheniformis* has a filamentous form colony morphology. Further optionally, the at least one strain of *Bacillus licheniformis* has a raised elevation colony morphology. Further optionally, the at least one strain of *Bacillus licheniformis* has a filamentous margin colony morphology.

Optionally, the at least one strain of *Bacillus licheniformis* comprises a gram positive, catalase positive and oxidase positive rod shaped strain that has a filamentous form colony morphology. Further optionally, the at least one strain of *Bacillus licheniformis* comprises a gram positive, catalase positive and oxidase positive rod shaped strain that has a white coloured, rough surfaced, filamentous form colony morphology.

Optionally, the at least one strain of *Bacillus licheniformis* comprises a gram positive, catalase positive and oxidase positive rod shaped strain that has a raised elevation colony morphology. Further optionally, the at least one strain of *Bacillus licheniformis* comprises a gram positive, catalase positive and oxidase positive rod shaped strain that has a white coloured, rough surfaced, raised elevation colony morphology.

Optionally, the at least one strain of *Bacillus licheniformis* comprises a gram positive, catalase positive and oxidase positive rod shaped strain that has a filamentous margin colony morphology. Further optionally, the at least one strain of *Bacillus licheniformis* comprises a gram positive, catalase positive and oxidase positive rod shaped strain that has a white coloured, rough surfaced, filamentous margin colony morphology.

Optionally, the at least one strain of *Bacillus licheniformis* comprises a strain selected from the group comprising:
(i) a *Bacillus licheniformis* strain having a white colour, rough surface, irregular form, flat elevation, and curled margin colony morphology;
(ii) a *Bacillus licheniformis* strain having a white colour, rough surface, filamentous form, raised elevation, and filamentous margin colony morphology; and
(iii) combinations thereof.

Optionally, the at least one strain of *Bacillus licheniformis* comprises a *Bacillus licheniformis* strain having a white colour, rough surface, irregular form, flat elevation, and curled margin colony morphology.

Optionally, the at least one strain of *Bacillus licheniformis* comprises a *Bacillus licheniformis* strain having a white colour, rough surface, filamentous form, raised elevation, and filamentous margin colony morphology.

Optionally, the bacterial composition comprises both:
(i) a *Bacillus licheniformis* strain having a white colour, rough surface, irregular form, flat elevation, and curled margin colony morphology; and
(ii) a *Bacillus licheniformis* strain having a white colour, rough surface, filamentous form, raised elevation, and filamentous margin colony morphology.

A bacterial composition according to the invention comprises or consists essentially of the *Bacillus licheniformis* strain deposited at NCIMB Ltd. with accession number NCIMB 42690.

Another bacterial composition according to the invention comprises or consists essentially of the *Bacillus licheniformis* strain deposited at NCIMB Ltd. with accession number NCIMB 42691.

A further bacterial composition according to the invention
comprises or consists of or consists essentially of both the *Bacillus licheniformis* strain deposited at NCIMB Ltd. with accession number NCIMB 42690, and the *Bacillus licheniformis* strain deposited at NCIMB Ltd. with accession number NCIMB 42691.

According to the present invention, there is provided a kit of parts defined in claim 3 for use in preparing augmented compost and/or farming, the kit of parts comprising the bacterial composition.

There is provided a kit of parts for use in preparing augmented compost and/or farming, the kit of parts comprising the bacterial composition defined in claim 5 and comprising a strain selected from the group comprising:
a. a *Bacillus licheniformis* strain with accession number NCIMB 42690 having a white colour, rough surface, irregular form flat elevation, and curled margin colony morphology;
b. a *Bacillus licheniformis* strain with accession number NCIMB 42691 having a white colour, rough surface, filamentous form raised elevation, and filamentous margin colony morphology; and
c. combinations thereof.

Thus, in one embodiment of the invention there is provided a kit of parts for use in preparing augmented compost and/or farming, the kit of parts in which the bacterial composition comprising a *Bacillus licheniformis* strain with accession number NCIMB 42690 having a white colour, rough surface, irregular form, flat elevation, and curled margin colony morphology.

Thus, in another embodiment of the invention there is provided a kit of parts for use in preparing augmented compost and/or farming, the kit of parts in which the bacterial composition comprising a *Bacillus licheniformis* strain with accession number NCIMB 42691 having a white colour, rough surface, filamentous form, raised elevation, and filamentous margin colony morphology.

Thus, in a further embodiment of the invention there is provided a kit of parts for use in preparing augmented compost and/or farming, the kit of parts in which the bacterial composition comprising both:
a. a *Bacillus licheniformis* strain with accession number NCIMB 42690 having a white colour, rough surface, irregular form flat elevation, and curled margin colony morphology; and
b. a *Bacillus licheniformis* strain with accession number NCIMB 42691 having a white colour, rough surface, filamentous form raised elevation, and filamentous margin colony morphology.

Thus, there is provided a kit of parts for use in preparing augmented compost and/or farming, the kit of parts in which the bacterial composition comprising the *Bacillus licheniformis* strain deposited at NCIMB Ltd. with accession number NCIMB 42690.

Thus, there is provided a kit of parts for use in preparing augmented compost and/or farming, the kit of parts in which the bacterial composition comprising the *Bacillus licheniformis* strain deposited at NCIMB Ltd. with accession number NCIMB 42691.

Thus, there is provided a kit of parts for use in preparing augmented compost and/or farming, the kit of parts in which the bacterial composition comprising the *Bacillus licheniformis* strain deposited at NCIMB Ltd. with accession number NCIMB 42690, and the *Bacillus licheniformis* strain deposited at NCIMB Ltd. with accession number NCIMB 42691.

Optionally, the kit of parts further comprises instructions for use thereof.

According to the present invention, there is provided a method for preparing augmented compost as defined in claim 5 the method comprising the steps of:
(i) providing the bacterial composition;
(ii) providing compost;
(iii) inoculating the compost with the bacterial composition according to the invention, optionally under temperature-controlled conditions, optionally under aerobic conditions.

Thus, the method of preparing augmented compost comprises the steps of:
(i) providing the bacterial composition comprising a strain selected from the in which:
   d. a *Bacillus licheniformis* strain with accession number NCIMB 42690 having a white colour, rough surface, irregular form flat elevation, and curled margin colony morphology;
   e. a *Bacillus licheniformis* strain with accession number NCIMB 42691 having a white colour, rough surface, filamentous form raised elevation, and filamentous margin colony morphology; and
   f. combinations thereof;
(ii) providing compost;
(iii) inoculating the compost with the bacterial composition, optionally under temperature-controlled conditions, optionally under aerobic conditions.

Thus, in one embodiment of the invention the method of preparing augmented compost comprises the steps of:
(i) providing the bacterial composition which comprises a *Bacillus licheniformis* strain with accession number NCIMB 42690 having a white colour, rough surface, irregular form, flat elevation, and curled margin colony morphology;
(ii) providing compost;
(iii) inoculating the compost with the bacterial composition, optionally under temperature-controlled conditions, optionally under aerobic conditions.

Thus, in another embodiment of the invention the method of preparing augmented compost comprises the steps of:
(i) providing the bacterial composition which comprises a *Bacillus licheniformis* strain with accession number NCIMB 42691 having a white colour, rough surface, filamentous form, raised elevation, and filamentous margin colony morphology; and
(ii) providing compost;
inoculating the compost with the bacterial composition, optionally under temperature-controlled conditions, optionally under aerobic conditions.

Thus, in a further embodiment of the invention the method of preparing augmented compost comprises the steps of:
(i) providing the bacterial composition which comprises both:
   c. a *Bacillus licheniformis* strain with accession number NCIMB 42690 having a white colour, rough surface, irregular form flat elevation, and curled margin colony morphology; and
   d. a *Bacillus licheniformis* strain with accession number NCIMB 42691 having a white colour, rough surface, filamentous form raised elevation, and filamentous margin colony morphology;
(ii) providing compost;
(iii) inoculating the compost with the bacterial composition, optionally under temperature-controlled conditions, optionally under aerobic conditions.

Thus, the method of preparing augmented compost comprises the steps of:
(i) providing the bacterial composition which comprises the *Bacillus licheniformis* strain deposited at NCIMB Ltd. with accession number NCIMB 42690;
(ii) providing compost;
(iii) inoculating the compost with the bacterial composition, optionally under temperature-controlled conditions, optionally under aerobic conditions.

Thus, the method of preparing augmented compost comprises the steps of:
(i) providing the bacterial composition which comprises the *Bacillus licheniformis* strain deposited at NCIMB Ltd. with accession number NCIMB 42691;
(ii) providing compost;
(iii) inoculating the compost with the bacterial composition, optionally under temperature-controlled conditions, optionally under aerobic conditions.

Thus, the method of preparing augmented compost comprises the steps of:
(i) providing the bacterial composition which comprises both the *Bacillus licheniformis* strain deposited at NCIMB Ltd. with accession number NCIMB 42690, and the *Bacillus licheniformis* strain deposited at NCIMB Ltd. with accession number NCIMB 42691;
(ii) providing compost;
(iii) inoculating the compost with the bacterial composition, optionally under temperature-controlled conditions, optionally under aerobic conditions.

Optionally, the method for preparing augmented compost is carried out under temperature-controlled conditions.

Optionally, the method for preparing augmented compost is carried out under aerobic conditions. Optionally, the method for preparing augmented compost is carried out under temperature-controlled and aerobic conditions.

Optionally, the step of inoculating the compost with the bacterial composition is carried out under temperature-controlled conditions.

Optionally, the step of inoculating the compost with the bacterial composition is carried out under aerobic conditions.

Optionally, the step of inoculating the compost with the bacterial composition is carried out under temperature-controlled and aerobic conditions.

According to the present invention, there is provided augmented compost prepared according to the method of preparing augmented compost.

According to the invention there is provided augmented compost prepared according to a method for preparing augmented compost, the method comprising the steps of:
(i) providing the bacterial composition which comprises a strain selected from the group comprising:
   a. a *Bacillus licheniformis* strain with accession number NCIMB 42690 having a white colour, rough surface, irregular form flat elevation, and curled margin colony morphology;
   b. a *Bacillus licheniformis* strain with accession number NCIMB 42691 having a white colour, rough surface, filamentous form raised elevation, and filamentous margin colony morphology; and
   c. combinations thereof;
(ii) providing compost;
inoculating the compost with the bacterial composition, optionally under temperature-controlled conditions, optionally under aerobic conditions.

In one embodiment of the invention there is provided augmented compost prepared according to a method for preparing augmented compost, the method comprising the steps of:
(i) providing the bacterial composition which comprises a *Bacillus licheniformis* strain with accession number NCIMB 42690 having a white colour, rough surface, irregular form, flat elevation, and curled margin colony morphology;
(ii) providing compost;
inoculating the compost with the bacterial composition, optionally under temperature-controlled conditions, optionally under aerobic conditions.

In another embodiment of the invention there is provided augmented compost prepared according to a method for preparing augmented compost, the method comprising the steps of:
(i) providing the bacterial composition which comprises a *Bacillus licheniformis* strain with accession number NCIMB 42691 having a white colour, rough surface, filamentous form, raised elevation, and filamentous margin colony morphology;
(ii) providing compost;
inoculating the compost with the bacterial composition, optionally under temperature-controlled conditions, optionally under aerobic conditions.

In a further embodiment of the invention there is provided augmented compost prepared according to a method for preparing augmented compost, the method comprising the steps of:
(i) providing the bacterial composition which comprises both:
   a. a *Bacillus licheniformis* strain with accession number NCIMB 42690 having a white colour, rough surface, irregular form flat elevation, and curled margin colony morphology; and
   b. a *Bacillus licheniformis* strain with accession number NCIMB 42691 having a white colour, rough surface, filamentous form raised elevation, and filamentous margin colony morphology;
(ii) providing compost;
(iii) inoculating the compost with the bacterial composition, optionally under temperature-controlled conditions, optionally under aerobic conditions.

Thus, there is provided augmented compost prepared according to a method for preparing augmented compost, the method comprising the steps of:
(i) providing the bacterial composition which comprises the *Bacillus licheniformis* strain deposited at NCIMB Ltd. with accession number NCIMB 42690;
(ii) providing compost;
(iii) inoculating the compost with the bacterial composition, optionally under temperature-controlled conditions, optionally under aerobic conditions.

Thus, there is provided augmented compost prepared according to a method for preparing augmented compost, the method comprising the steps of:
(i) providing the bacterial composition which comprises the *Bacillus licheniformis* strain deposited at NCIMB Ltd. with accession number NCIMB 42691;
(ii) providing compost;
(iii) inoculating the compost with the bacterial composition, optionally under temperature-controlled conditions, optionally under aerobic conditions.

Thus, there is provided augmented compost prepared according to a method for preparing augmented compost, the method comprising the steps of:
(i) providing the bacterial composition which comprises both the *Bacillus licheniformis* strain deposited at NCIMB Ltd. with accession number NCIMB 42690, and the *Bacillus licheniformis* strain deposited at NCIMB Ltd. with accession number NCIMB 42691;
(ii) providing compost;
(iii) inoculating the compost with the bacterial composition, optionally under temperature-controlled conditions, optionally under aerobic conditions.

Optionally, the augmented compost is prepared under temperature-controlled conditions.

Optionally, the augmented compost is prepared under aerobic conditions.

Optionally, the augmented compost is prepared under temperature-controlled and aerobic conditions.

Optionally, the augmented compost is prepared according to a method for preparing augmented compost comprising the step of inoculating the compost with the bacterial composition under temperature-controlled conditions.

Optionally, the augmented compost is prepared according to a method for preparing augmented compost comprising the step of inoculating the compost with the bacterial composition is carried out under aerobic conditions.

Optionally, the augmented compost is prepared according to a method for preparing augmented compost comprising the step of inoculating the compost with the bacterial composition is carried out under temperature-controlled and aerobic conditions.

According to the present invention, there is provided augmented compost comprising the bacterial composition as defined in in claim 8.

According to the invention there is provided augmented compost comprising the bacterial composition comprising a strain selected from the group which comprises:
a. a *Bacillus licheniformis* strain having a white colour, rough surface, irregular form, flat elevation, and curled margin colony morphology;
b. a *Bacillus licheniformis* strain having a white colour, rough surface, filamentous form, raised elevation, and filamentous margin colony morphology; and
c. combinations thereof.

In an embodiment of the invention there is provided augmented compost comprising the bacterial composition comprising a *Bacillus licheniformis* strain with accession number NCIMB 42690 having a white colour, rough surface, irregular form, flat elevation, and curled margin colony morphology;

In another embodiment of to the invention there is provided augmented compost comprising the bacterial composition comprising a *Bacillus licheniformis* strain with accession number NCIMB 42691 having a white colour, rough surface, filamentous form, raised elevation and filamentous margin colony morphology.

In a further embodiment of the invention there is provided augmented compost comprising the bacterial composition comprising both:
a. a *Bacillus licheniformis* strain with accession number NCIMB 42690 having a white colour, rough surface, irregular form flat elevation, and curled margin colony morphology; and
b. a *Bacillus licheniformis* strain with accession number NCIMB 42691 having a white colour, rough surface, filamentous form raised elevation, and filamentous margin colony morphology.

Thus, there is provided augmented compost comprising the bacterial composition comprising a strain selected from the group comprising the *Bacillus licheniformis* strain deposited at NCIMB Ltd. with accession number NCIMB 42690.

Thus, there is provided augmented compost comprising the bacterial composition comprising the *Bacillus licheniformis* strain deposited at NCIMB Ltd. with accession number NCIMB 42691.

Thus, there is provided augmented compost comprising the bacterial composition comprising both the Bacillus licheniformis strain deposited at NCIMB Ltd. with accession number NCIMB 42690, and the Bacillus licheniformis strain deposited at NCIMB Ltd. with accession number NCIMB 42691.

According to the present invention, there is provided a use of augmented compost as defined in claim 9 comprising the bacterial composition according to the invention as a nutrient source for farming, the use comprising the steps of:
(i) providing the augmented compost;
(ii) providing at least one fungus, mycelium, spawn, spore, plant, cutting, turf, and/or seed;
(iii) cultivating the at least one fungus, mycelium, spawn, spore, plant, cutting, turf, and/or seed in an environment comprising the augmented compost.

In one embodiment of the invention there is provided a use of augmented compost comprising the bacterial composition as a nutrient source for fungus farming, the use comprising the steps of:
(i) providing the augmented compost;
(ii) providing at least one fungus, mycelium, spawn, and/or spore;
(iii) cultivating the at least one fungus, mycelium, spawn, and/or spore, in an environment comprising the augmented compost.

In a another embodiment of the invention there is provided a use of augmented compost comprising the bacterial composition as a nutrient source for mushroom farming, the use comprising the steps of:
(i) providing the augmented compost;
(ii) providing at least one mushroom, mycelium, spawn, and/or spore;
(iii) cultivating the at least one mushroom, mycelium, spawn, and/or spore, in an environment comprising the augmented compost.

In a further embodiment of the invention there is provided a use of augmented compost comprising the bacterial composition as a nutrient source for *Agaricus bisporus* farming, the use comprising the steps of:
(i) providing the augmented compost;
(ii) providing at least one *Agaricus bisporus* mushroom, mycelium, spawn, and/or spore;
(iii) cultivating the at least one mushroom, mycelium, spawn, and/or spore, in an environment comprising the augmented compost.

Thus, there is provided a use of augmented compost according to the invention as a nutrient source for fungus farming, the use comprising the steps of:
(iv) providing the augmented compost;
(v) providing at least one fungus, mycelium, spawn, and/or spore;
(vi) cultivating the at least one fungus, mycelium, spawn, and/or spore, in an environment comprising the augmented compost.

Thus, there is provided a use of augmented compost according to the invention as a nutrient source for mushroom farming, the use comprising the steps of:
(iv) providing the augmented compost;
(v) providing at least one mushroom, mycelium, spawn, and/or spore;
(vi) cultivating the at least one mushroom, mycelium, spawn, and/or spore, in an environment comprising the augmented compost.

Thus, there is provided a use of augmented compost according to the invention as a nutrient source for *Agaricus bisporus* farming, the use comprising the steps of:
(iv) providing the augmented compost;
(v) providing at least one *Agaricus bisporus* mushroom, mycelium, spawn, and/or spore;
(vi) cultivating the at least one mushroom, mycelium, spawn, and/or spore, in an environment comprising the augmented compost.

For the avoidance of doubt, MM76 refers to the strain deposited under accession number NCIMB 42690. Likewise, MM172 refers to the strain deposited under accession number NCIMB 42691.

### Brief description of the drawings

**Figure 1** **parts (A) and (B) each** shows an image of the colonies formed by MM76 on TSA media.
**Figure 2** **parts (A) and (B) each** shows an image of the colonies formed by MM172 on TSA media.
**Figure 3** shows a chart representing typical glucanase activity in control and augmented compost at day 2 and day 7 of composting at bench scale.
**Figure 4** shows charts representing the two typical scenarios observed among the different trials performed. The simple sugar production in augmented compared to control compost prepared in the bench composting system showed that: a) the levels of glucose increased significantly, but the levels of trehalose (a biosynthetic product of glucose) did not; b) the levels of glucose did not rise detectably, but the levels of trehalose were increased. This suggests that in scenario a) the compost was sampled slightly earlier in the degradation of cellulose than in scenario b).
**Figure 5** shows a chart representing laccase activity of both control and augmented compost during colonisation by *Agaricus bisporus* mycelium at bench scale. Results presented are an average of triplicates from three trials.
**Figure 6** shows charts representing laccase activities in control and augmented composts during colonisation by *Agaricus bisporus* mycelium throughout (A) Phase 3 composting and (B) during case run. Results presented are an average of triplicates from two commercial scale trials.
**Figure 7** shows a chart representing the percentage increase in mushrooms yield from the augmented compost compared to control, indicating that the highest increases in yield were predominantly from flushes 2 and 3.
**Figure 8** shows a chart representing mushroom piece weight according the cup diameter.

### Detailed description of the invention

The inventors have isolated 389 bacterial and 210 fungal strains from compost at different stages of production and from different compost yards in Ireland, based in Co. Monaghan and Co. Kildare, respectively. All of the bacterial strains were identified and assessed for their ability to breakdown complex carbohydrates that are associated with mushroom compost (cellulose, hemicellulose and lignin). Several that scored highly were selected for further assessment of their effect in the bioaugmentation of compost, in terms of improving compost productivity.

Small microcosm and larger semi- commercial scale trials were used to confirm the beneficial effects of these on compost quality. The compost from the semi- commercial scale trials was used to grow mushrooms in a trial mushroom farm, confirming that the augmented compost consistently yielded 4% higher than un-augmented compost. Additionally, the mushrooms cultivated from augmented compost had a higher piece weight (3-5%) than those from control compost. The results of these trials were consistent and were observed in compost produced during different seasons and in different locations. It was concluded that the augmentation of compost with the MM76/MM 172 microbial composition led to an increase in soluble sugars, promoting the colonisation of compost by *Agaricus bisporus.* This resulted in a higher yield and quality of mushrooms.

Compost for the cultivation of *Agaricus bisporus* is composed of complex carbohydrates such as cellulose, hemicellulose and lignin. These are gradually broken down into simple sugars during the composting process, through the activity of the microbial community within the compost. These complex carbohydrates are further broken down during the mushroom cultivation process.

One mushroom cropping cycle involves the production of three mushrooms flushes. However, the yield from each mushroom flush decreases significantly. There have been many hypotheses put forward to explain this successive reduction in mushroom yield, including a decrease in the microbial breakdown of complex carbohydrates.

In order to address this hypothesis, the inventors have developed a composition comprising at least one of two *Bacillus licheniformis* strains (strain MM76 (deposited under accession number NCIMB 42690) and/or strain MM172 (deposited under accession number: NCIMB 42691)); each strain was originally isolated from mushroom compost. It was demonstrated that the augmentation of compost with this microbial composition increased the cellulase activity, leading to the increased production of glucose and/ or trehalose. The augmented compost, with the higher levels of soluble sugars, was better colonised with *Agaricus bisporus* mycelium and this consistently led to a higher yield of heavier mushrooms.

The composition could comprise strain MM76 alone, or strain MM172 alone, or both strains MM76 and MM172. Any such composition, comprising at least one of strain MM76 and/or strain MM172, is able to degrade complex carbohydrates, and/or to degrade protein, and/or to assimilate ammonia. Addition of such a composition comprising at least one of strain MM76 and/or strain MM172 to compost led to increased production of soluble sugars such as glucose and/or trehalose from complex carbohydrates within the compost. Addition of such a composition comprising at least one of strain MM76 and/or strain MM172 to compost further led to better colonisation of the augmented compost with *Agaricus bisporus* mycelium. Addition of such a composition comprising at least one of strain MM76 and/or strain MM172 to compost further led to a higher yield of heavier mushrooms.

A composition comprising just one of strain MM76 or strain MM172 is effective, while a composition comprising both strain MM76 and strain MM172 is also effective. The two strains MM76 and MM172 do not inhibit each other. In particular, neither strain MM76 nor strain MM172 significantly inhibited the growth of the other strain. On the contrary, in a composition comprising both strain MM76 and strain MM172, the strains have a synergistic effect. We postulate that the synergistic effect is related to the different optimal growing temperature of the two strains. The optimal temperature range for the growth of the strain MM76 only partially overlaps that for the strain MM172. The combination of the optimal temperature ranges for the growth of both isolates covers the entire temperature range of the industrial process to produce the augmented compost.

While the present invention has application in mushroom farming, in particular farming of *Agaricus bisporus,* is clear that a composition comprising at least one of strain MM76 and/or strain MM172 could be usefully added to compost, soil, fertilizer or another fungus or plant farming medium or growth medium; to improve the farming of any of a wide variety of fungi and/or plants, including for example edible mushrooms, button mushrooms (*Agaricus bisporus*)*,* Portobello mushrooms (*Agaricus bisporus*)*,* straw mushrooms (*Volvariella volvacea*)*,* oyster mushrooms (*Pleurotus ostreatus*)*,* shiitakes (*Lentinula edodes*)*,* enokitake (*Flammulina spp.*)*,* fruit, bananas, apples, pears, oranges, melons, strawberries, nuts, vegetables, potato, carrot, legume pulses, cassava, groundnuts, beans, peas, green vegetables, broccoli, salad vegetables, celery, lettuce, sweet potatoes, grasses, rice, wheat, sugarcane, maize, corn, soybean, sunflower rapeseed, mustard. Furthermore, it is clear that a composition comprising at least one of strain MM76 and/or strain MM172 could be used to augment, or in the preparation of, compost soil, fertilizer or another fungus or plant farming medium for sale. Furthermore, it is clear that a composition comprising at least one of strain MM76 and/or strain MM172 could be used in Mycoremediation. Furthermore, it is clear that a composition comprising at least one of strain MM76 and/or strain MM172 could be used in food/ingredient preparation and/or in food technology.

One of these two *Bacillus licheniformis* strains, referred to as MM76, was determined to be a gram positive, catalase positive and oxidase positive rod shaped bacillus. Sequencing of the 16S ribosomal RNA (SEQ ID No. 3) indicated that MM76 was a *Bacillus licheniformis* strain. On the 16^{th} November 2016, MM76 was deposited under the Budapest Treaty in NCIMB and was confirmed as a *Bacillus licheniformis* strain (Accession number: NCIMB 42690).

The other of these two *Bacillus licheniformis* strains, referred to as MM172 was determined to be a gram positive, catalase positive and oxidase positive rod shaped bacillus. Sequencing of the 16S ribosomal RNA (SEQ ID No. 4) indicated that MM172 was a *Bacillus licheniformis* strain. On the 16^{th} November 2016, MM172 was deposited under the Budapest Treaty in NCIMB and was confirmed as a *Bacillus licheniformis* strain (Accession number: NCIMB 42691).

### Examples

### Isolation of bacteria from compost

Isolation of bacteria from compost was as follows: 10g of Phase 1 mushroom compost was added to 90 ml of Maximum Recovery Diluent (MRD) and agitated for 30 mins at 150 rpm. Serial dilutions were prepared and spread onto different media agar plates. These were incubated at 25°C, 37°C and 55°C for 24-48 hrs and any colonies that appeared on the plates were sub-cultured. Pure colonies were stored as 50% glycerol stocks at -80°C for further analysis.

Catalase and oxidase activities were assayed using hydrogen peroxide breakdown and tetramethyl- or dimethyl p-phenlyenediamine. Gram characterisation was conducted using a commercially available Gram staining kit.

### Purification and amplification of DNA

Total genomic DNA was extracted from bacterial isolates using a commercial genomic DNA extraction kit (such as the Invitrogen PureLink^{®} Genomic DNA kit) according to the manufacturer's instructions. PCR amplification of the 16S rRNA gene was conducted for sequence identification of bacterial isolates. The DNA fragments were amplified using the primers 27f (5'-AGAGTTTGGATCMTGGCTCAG-3') (SEQ ID NO: 1) and 1492r (5'-CGGTTACCTTGTTACGACTT-3') (SEQ ID NO: 2) (Jiang *et al.,* 2006). The PCR was performed using a commercial high-fidelity PCR kit (such as the Q5^{®} Hot Start High Fidelity master mix; New England Biolabs^{®} Inc.), in a final volume of 25µl per reaction. The PCR condition used were the following: initial denaturation at 98°C for 60 seconds; 35 cycles of denaturation (98°C, 10 seconds), annealing (55°C, 20 seconds) and DNA extension (72°C, 35 seconds); final DNA extension at 72°C for 2 minutes. The amplicons obtained were sequenced both in forward and reverse. This resulted in a - 1.5 Kbp long consensus sequence being obtained for each isolate. The sequences were queried using an online BLAST^{®} nucleotide database (for example on the website of the National Center for Biotechnology Information of the US National Library of Medicine available at https://blast.ncbi.nlm.nih.gov/Blast.cgi) and identifications were considered reliable for results matching >98% of identity.

### In vitro assessment of the ability of bacterial strains to degrade complex carbohydrates

The 389 bacterial isolates were tested for their ability to degrade four different carbon sources: hemicellulose (growth medium: 5g/L xylan agar+ 1mg/ml congo red stain after bacterial growth); proteins (growth medium: 15g/L skim milk agar); cellulose (growth medium: 25g/L carboxymethil-cellulose agar + 1mg/ml congo red stain after bacterial growth); lignin (growth medium: Luria-Bertani amended with congo red/ toluidine blue agars). The isolates were also tested for their ability to assimilate ammonia (growth medium: glucose, NH₄Cl, ferric citrate, NaCl, MgSO₄, MnSO₄, K₂HPO₄, KH₂PO₄ agar).

Cultures were incubated at the optimal temperatures (25°C for the low mesophiles; 37°C for the mesophiles; 55°C for the thermophiles) for 48 hours. For lignin and ammonia assays, colony growth was used as a parameter to estimate the lignin/ ammonia utilisation. For cellulose and hemicellulose assays, the presence and the dimension of halos around the colonies was observed after washing the medium with the congo red solution, followed by a medium wash with 5M NaCl solution. For protein assay, the presence and the dimension of a halo around the bacterial colony was used to estimate the protein assimilation. The higher the colony/ halo dimension, the higher the score assigned, ranging from 0 (no growth/ halo) to +++ (large colony/ halo). The score assignment was qualitative and subjective. The total score was used to develop a numerical rating to easily identify those microbes with the greatest abilities to degrade the complex carbohydrates (0 -18). The arbitrary threshold chosen to identify the potential candidates for inclusion in a microbial composition was 15.

### Preparation of the bacterial strains for use in small and large scale composting trials.

Bacterial cultures were prepared using commercially available bench-scale bioreactor/fermenters system (such as Eppendorf BioFlo 120 (10L) fermenters). Strain MM76 was fermented in Tryptic Soy Broth (TSB) at 55°C at 25% air saturation until the end of exponential phase (~6.5hrs), resulting in 3x10⁹ viable cells/ ml. Strain MM172 was fermented in Tryptic Soy Broth (TSB) at 37°C at 25% air saturation until the end of exponential phase (~6.5hrs), resulting in 1.5×10⁹ viable cells/ ml.

### Small scale assessment of the ability of the bacterial strains to degrade complex carbohydrates during mushroom composting

The small scale assessment of the ability of the MM76/MM172 composition to increase the productivity of compost was conducted using a bench composting system (BCS) that can be used to produce mushroom compost. This system was set up following the specifications described in Noble *et al.* (1997), who performed the entire mushroom composting process in 2L flasks. Those specifications were adapted to the upscaled system and adjusted until optimal results were reached. These included an initial warming up stage, to bring the temperature up to 60°C for 12-18 hours, followed by a pasteurisation stage at temperatures between 44-48°C and a final conditioning stage at 25°C for 24 hours (Colak, 2004). 2.6kg of the starter mushroom compost was filled into a 10L culture vessel and then placed into a temperature-controlled stainless steel water bath. Prior to filling, a stainless steel platform was placed in the bottom of each flask to allow air to be driven up through the compost. The stainless steel platforms were perforated with 2mm holes to allow the air to pass through them. Rubber tubing, which is connected to an air-flow meter, was inserted through a central hole in the platform. The temperature in the waterbath was manually changed to reach the temperature profile needed for the process. The air flow meter was used to ensure a constant air flow through the compost, to provide aerobic conditions. Vessel lids opening was managed to obtain the optimal moisture loss. A dissolved oxygen (DO)/temperature probe was inserted into the centre of the compost inside the vessel. The dissolved oxygen and temperature of the compost were measured and logged on a laptop using dedicated software. The dissolved oxygen profile was used to estimate if the proper aerobic fermentation occurred in the early composting stage, before the pasteurisation started. The composting conditions used mimicked those of the current industrial standards (Colak, 2004).

The bacterial cells, for example cells of strain MM76 and/or strain MM172, were re-suspended in a 0.9% NaCl sterile solution and applied to the compost using a sterile spray bottle. The compost was thoroughly mixed prior to filling the vessels, to ensure an even distribution of the microbial inoculum. Non-inoculated compost was sprayed with sterile 0.9% NaCl sterile solution, as a negative control.

Compost from inoculated and control vessels was sampled at day 2 and day 7 of the bench scale process for analysis of glucanase and xylanase activity. The assays were conducted by the spectrophotometric estimation of simple sugars (glucose, xylan) from the crude compost extract. This was determined by comparison with omologues sugars released by the digestion of commercial oat β-gtucan and beechwood xylan at known concentrations, according to the methodology described in King *et al.,* 2009. At the end of the process, the resultant composts were analysed for the water soluble sugar content using a high-performance anion exchange chromatography with pulsed amperometric detection (HPAEC-PAD) methodology. The high performance anion-exchange chromatography was performed on a commercially available high-performance anion exchange chromatography (HPAEC) system (such as Dionex^{®} ICS 5000+ system (Thermo Scientific^{®})) equipped with a suitable analytical column (such as CarboPac^{®} PA-1 column) in combination with a suitable guard column (such as CarboPac^{®} guard column) and pulsed amperometric detection (PAD). The elution of sugars was performed at a flow rate of 0.75 mL min-1, combining two mobile phases: (A) 0.2 M NaOH, (B) H₂O. Water soluble sugars were extracted from freeze-dried, grinded compost samples in 50% methanol at 70°C for 15 minutes. After centrifugation at 14000 rpm for 15 minutes, the supernatants were mixed with double-distilled water (ddH₂O). A further 1:2 H₂O dilution step was carried out, before 400 µL of the sample was filtered through a 0.2 µm nylon syringe filter and injected on to the HPAEC system.

### Small scale assessment of the ability of the augmented mushroom compost to be colonised by Agaricus bisporus mycelium.

To confirm if the compost produced by the BCS could be colonised by *Agaricus bisporus,* 4 spawn grains of a known *Agaricus bisporus* strain (such as Sylvan^{®} A15 strain spawn grains) were added to each 150 mm plate containing 50g of the control or augmented compost. The plates were left at room temperature for 21 days to allow the mycelium to colonise the compost. Laccase enzyme activity analysis was performed comparing the reduction of ABTS (2,2'-Azino-bis(3-ethylbenzothiazoline-6-sulfonic acid) by the compost crude extract with that obtained from a known concentration of a commercial laccase enzyme, followed by spectrophotometry analysis, according to the methodology described in Johannes and Majcherczyk, 2000.

### Large scale assessment of the ability of the bacterial strains to degrade complex carbohydrates during mushroom composting.

The freeze dried inoculum was re-suspended in 60L of tap water and left to re-hydrate for 60 minutes, with occasional stirring. 140 tonnes of compost were inoculated, ensuring a thorough mixing and an even distribution of the inoculum throughout the compost. The Phase 2 compost was spawned with a known *Agaricus bisporus* strain (such as Sylvan^{®} A15 strain) before being filled into a Phase 3 tunnel and the colonisation of the compost was allowed to proceed for 15-16 days. Compost samples were taken at the end of Phase 1, Phase 2 and Phase 3 to assess the moisture content, pH and the rate of mycelial colonisation (laccase activity, this assay was performed only on Phase 3 compost).

### Growing trials to confirm effect of compost augmentation on mushroom yield and quality

9 tonnes of the augmented compost and 9 tonnes of the control compost were each cased with mushroom casing on day 1. Initially, the CO₂ ranged between 8,000-12,000 ppm, temperature between 24-27°C and high humidity (>90%) (stimulating the ideal conditions for vegetative growth). Once an optimal casing colonisation was obtained, conditions were changed to stimulate the development of the fruiting body. Specifically, the temperature was reduced to a range of 17-22°C and the CO₂ to <2,000 ppm. A commercially available computer system (such as Fancom^{®}) was used to regulate the CO₂, air temperature and humidity in the growth house. The compost temperature was monitored throughout the cropping cycle, to estimate the difference in the total microbial activity between the augmented and the control compost. The laccase activity of compost was monitored on a daily basis from day 12 of Phase 3 to the end of case run. 10g of compost was mixed with distilled water and agitated for 20 minutes. The supernatant was collected and analysed as previously described. A higher laccase activity was correlated to a higher mushroom mycelium growth, and was generally in the range of 100-200 U/g_{DM} (units per gram of dry matter) at the end of Phase 3. The cropping cycle lasted 39 days and mushrooms from three flushes were assessed for yield (kg of mushrooms/ m² of compost) and quality (dry matter content, piece weight and cup diameter).

### Example 1: Isolation, identification and characterisation of Bacillus licheniformis strains MM76 and MM172

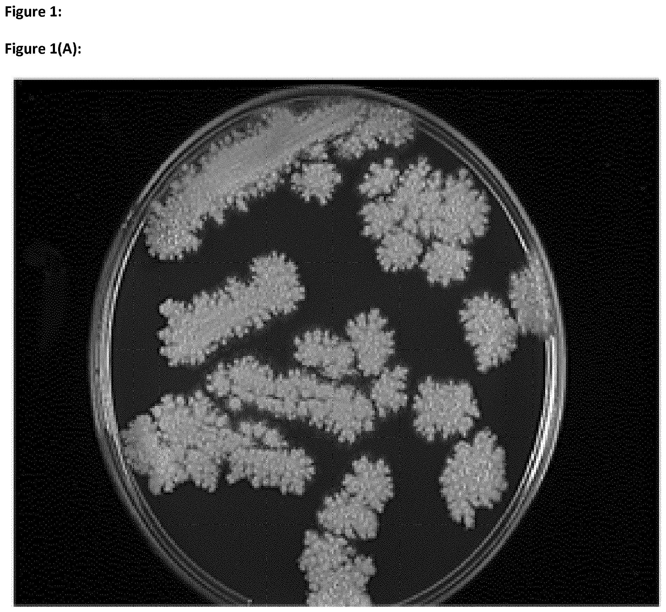

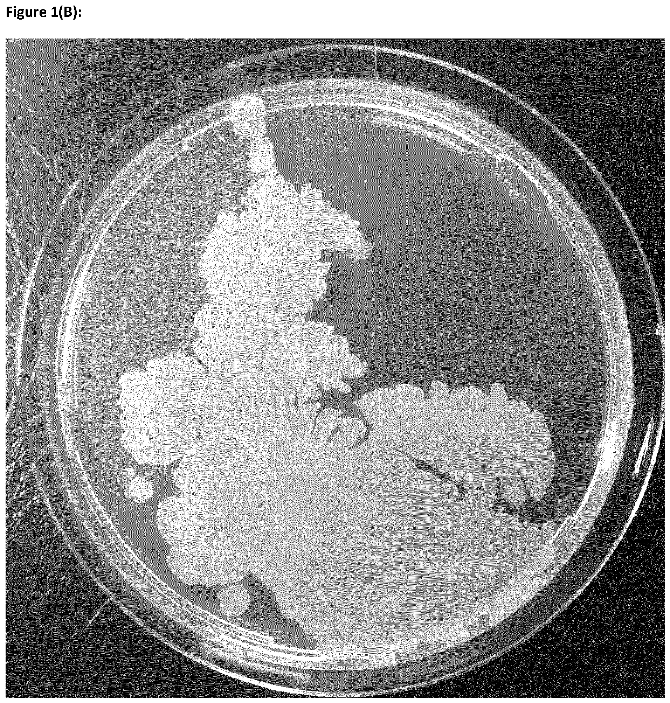

Strain MM76 was isolated using trypticase soy agar (TSA) media plates, incubated at 55°C for 24 hrs, from Phase 1 mushroom compost (composed of fermented wheat straw, chicken litter) from Kabeyun Compost, Co. Monaghan, Ireland. Individual colonies were re-streaked onto TSA media plates to obtain pure bacterial cultures. The colony and cellular morphology of MM76 were assessed (Figure 1) and MM76 strain was determined to be a gram positive, catalase positive and oxidase positive rod shaped bacillus, as summarised below in Table 1. Sequencing of the 16S ribosomal RNA indicated that MM76 was a *Bacillus licheniformis* strain. On the 16^{th} November 2016, MM76 was deposited under the Budapest Treaty in NCIMB and was confirmed as a *Bacillus licheniformis* strain (Accession number: NCIMB 42690).

**Table 1:**

| ***COLONY MORPHOLOGY*** | |
|---|---|
| Colour | White |
| Surface | Rough |
| Form | Irregular |
| Elevation | Flat |
| Margin | Curled |

| ***CELL MORPHOLOGY*** | |
|---|---|
| Cell shape | Rods |
| Other | Short bacilli, with filaments |

| ***BIOCHEMICAL CHARACTERISATION*** | |
|---|---|
| Catalase | POS |
| Oxidase | POS |
| Gram Stain | POS |

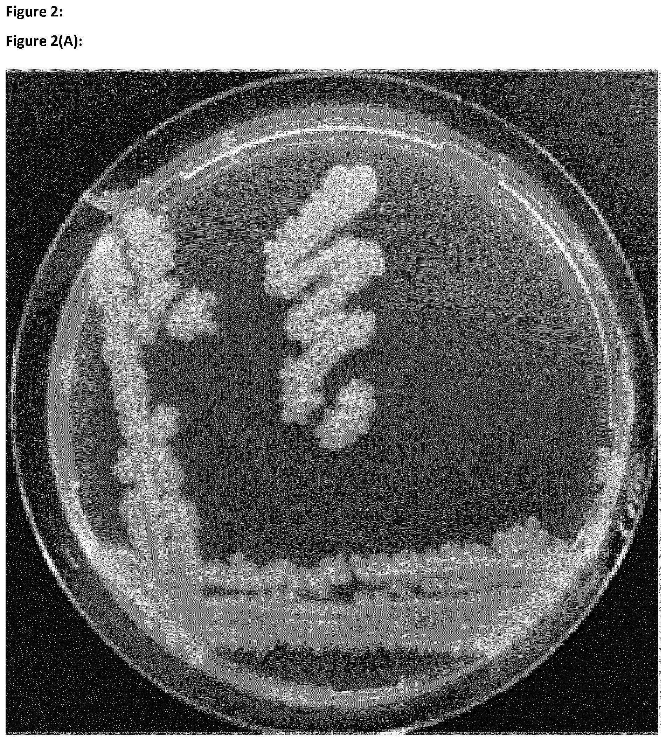

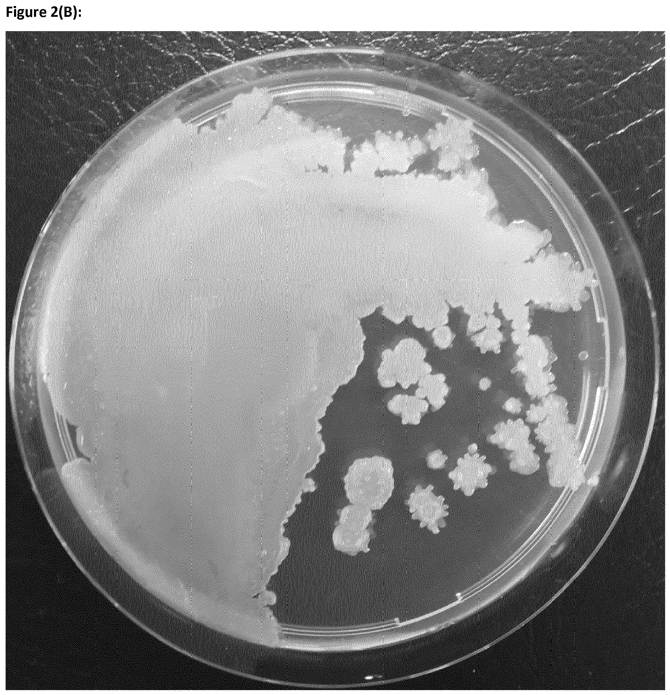

Strain MM172 was isolated using Actinomycete Isolation Agar (AA) media plates, incubated at 37°C for 24 hrs, from Phase 1 compost (composed of fermented wheat straw and chicken litter) from a bunker at Carbury Compost, Co. Kildare, Ireland. Individual colonies were re-streaked onto TSA plates to obtain pure bacterial cultures. The colony and cellular morphology of MM172 were assessed (Figure 2) and it was determined to be a gram positive, catalase positive and oxidase positive rod shaped bacillus, as summarised below in Table 2. Sequencing of the 16S ribosomal RNA indicated that MM172 was a *Bacillus licheniformis* strain. On the 16^{th} November 2016, MM172 was deposited under the Budapest Treaty in NCIMB and was confirmed as a *Bacillus licheniformis* strain (Accession number: NCIMB 42691).

**Table 2:**

| ***COLONY MORPHOLOGY*** | |
|---|---|
| Colour | White |
| Surface | Rough |
| Form | Filamentous |
| Elevation | Raised |
| Margin | Filamentous |

| ***CELL MORPHOLOGY*** | |
|---|---|
| Cell shape | Rods |

| ***BIOCHEMICAL CHARACTERISATION*** | |
|---|---|
| Catalase | POS |
| Oxidase | POS |
| Gram stain | POS |

### Example 2: In vitro assessment of the ability of Bacillus licheniformis strains MM76 and MM172 to degrade complex carbohydrates in mushroom compost

MM76 was the best thermophilic candidate for a microbial composition, achieving the highest results in all of the functional tests, with the exception of the hemicellulose test, as shown in Table 3. The mesophilic MM172 also achieved high scores in the majority of the functional tests, with the exception of the hemicellulose degradation and ammonia assimilation tests, as shown in Table 3. The high scoring of MM76 and MM172 in the *in vitro* tests resulted in their inclusion in the microbial composition. An *in vitro* assay was conducted to test if the growth of either isolate inhibited the other, and confirmed that although there was limited (non-significant) inhibition in one of the 6 tested media, there was no inhibition in the majority of tested media environments. The results are shown in Table 4.

**Table 3:**

| **Isolate** | **Lignin** | | **Hemicellulose** | **Cellulose** | **Protein** | **Ammonia** | **Total** | **Species** |
|---|---|---|---|---|---|---|---|---|
| | LB+CR | LB+TB | XY+CR | CMC+CR | SM | AA | | |
| MM76 | +++ | +++ | ++ | +++ | +++ | +++ | 17 | *Bacillus licheniformis* |
| MM172 | +++ | +++ | ++ | +++ | +++ | ++ | 16 | *Bacillus licheniformis* |

| | | | | | | | | |
|---|---|---|---|---|---|---|---|---|
| LB+CR: Luria Bertani Agar medium stained with Congo Red LB+TB: Luria Bertani Agar medium stained with Toluidine Blue XY+CR: Xylan Agar medium flooded with Congo Red CMC+CR: Carboxymethylcellulose Agar medium flooded with Congo Red SM: Skim Milk Agar AA: Ammonia Agar | | | | | | | | |

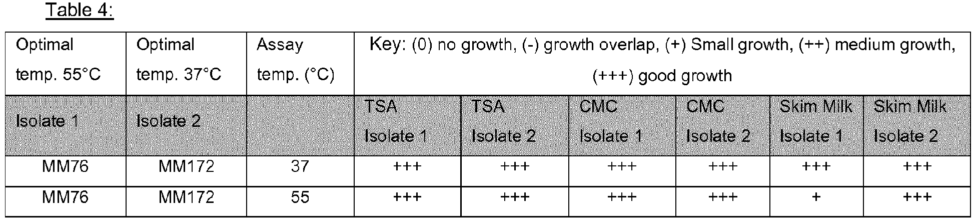

### Example 3: In situ assessment of the ability of Bacillus licheniformis strains MM172 and MM76 to degrade complex carbohydrates in mushroom compost

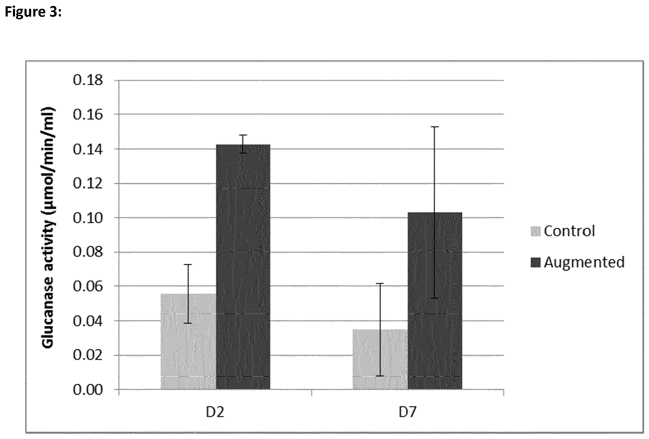

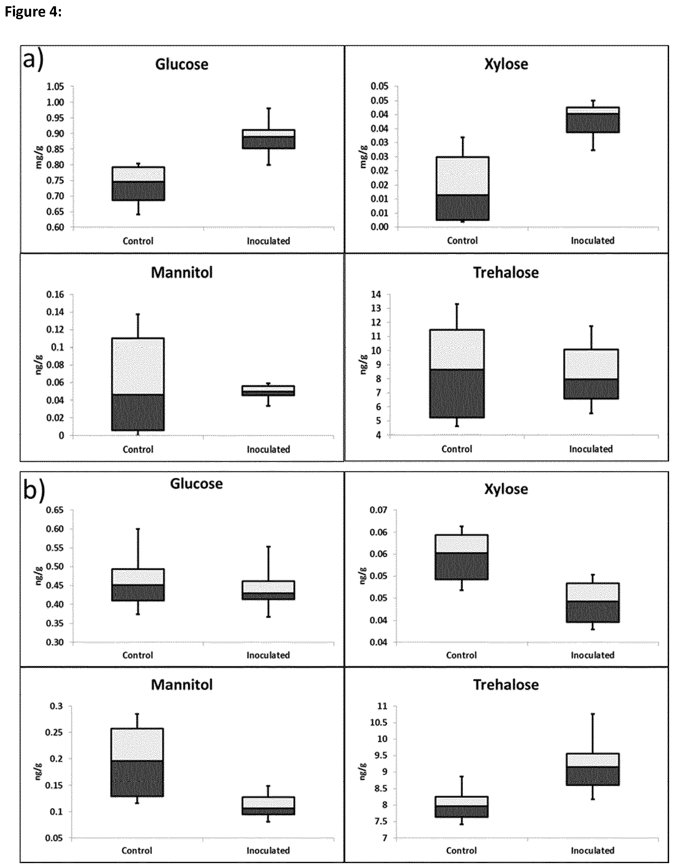

Control and augmented compost produced using the bench composting system was assessed for its level of complex carbohydrate degradation. The augmented compost comprised strains MM172 and/or MM76. Glucanase activity measured on day 2 and day 7 of the composting process confirmed that the augmented compost had higher levels of cellulose degrading activity than the control compost (Figure 3). This resulted in higher levels of the breakdown products of cellulose, glucose and trehalose (Figure 4).

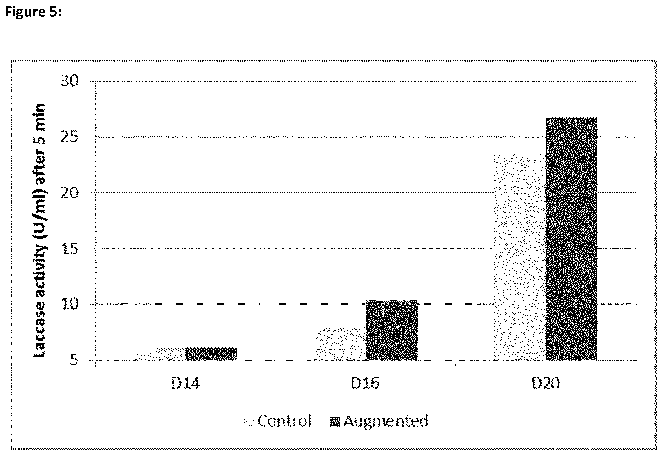

Mycelial colonisation assays were used to confirm that the compost produced using the BCS could be colonised by *Agaricus bisporus* over 21 days. Laccase is an enzyme that is produced by *Agaricus bisporus* in order to release simple carbohydrates. It has been reported to be a good indication of the success of the growth of the mushroom mycelium. Samples of compost were taken from replicate plates (containing 15g of compost) for analysis of laccase activity (Figure 5). At day 14, the laccase activity was similar in both control and augmented compost. However, on days 16- 20, the laccase activity was consistently highest in the augmented compost. This suggested that the rate of mycelial colonisation was quicker in the augmented compost compared to the control composts.

### Example 4: Large scale trials to confirm the efficacy of Bacillus licheniformis strains MM76 and MM172 to degrade complex carbohydrates in mushroom compost

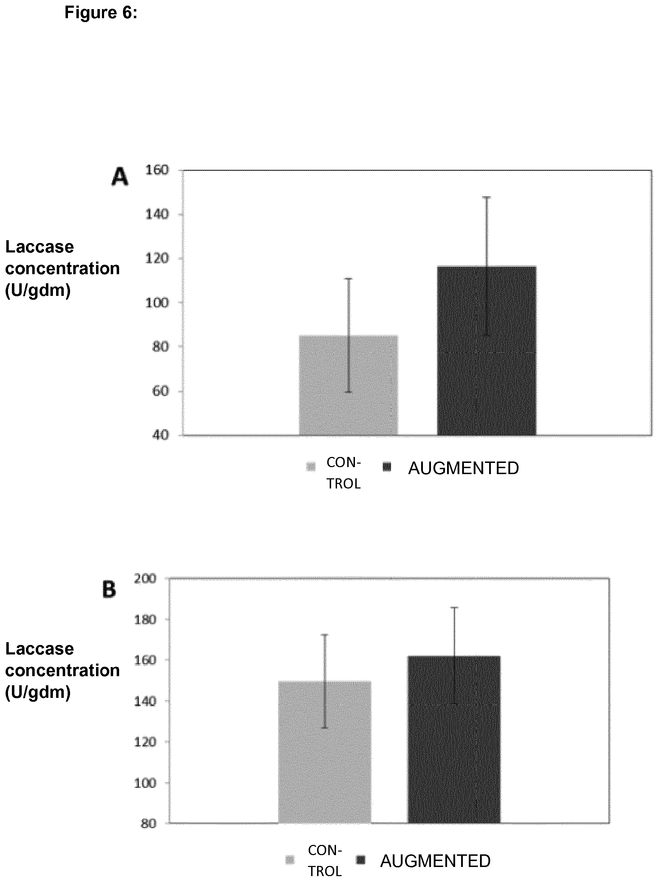

Quantification of the compost laccase activity was conducted from day 12 of Phase 3 composting to the end of case run, for a total of 8 days. Results confirmed that this enzyme activity was approximately 10% higher in the inoculated compost throughout this time period, indicating better mycelial colonisation of the compost (Figure 6).

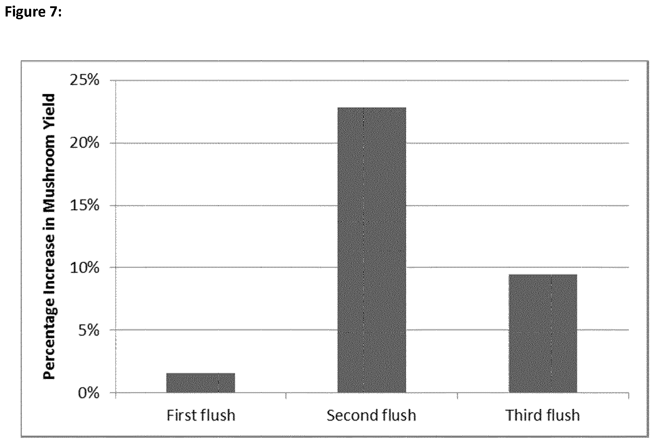

Mushrooms were harvested from each of three flushes and the yield was assessed (Figure 7). The augmented compost yielded approximately 8% higher than the control compost, with the majority of the increase observed in second flush.

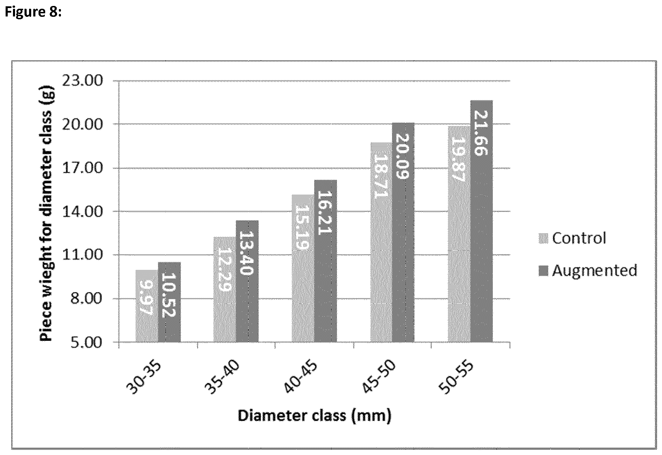

Piece weight and cup diameter of mushroom samples were measured (Figure 8). Mushrooms belonging to the "closed cup" category (class diameter ranging between 30-55 mm) from augmented compost were heavier than control. The increase in piece weight of mushrooms cultivated on augmented compost was 5-9% compared to those cultivated on control compost.

### References

Colak, M. 2004. Temperature profiles of Agaricus bisporus in composting stages and effects of different composts formulas and casing materials on yield. African Journal of Biotechnology, Vol. 3 (9), pp. 456-462.

Jiang, H., Dong, H., Zhang, G., Yu, B., Chapman, L.R. and Fields, M.W. 2006. Microbial diversity in water and sediment of Lake Chaka, an athalassohaline lake in northwestern China. Applied and Environmental Microbiology, Vol. 72 (6), pp. 3832-3845.

Johannes, C. and Majcherczyk, A. 2000. Laccase activity tests and laccase inhibitors. Journal of Biotechnology, Vol. 78 (2), pp. 193-199.

King, Brian C., Donnelly, Maria K., Bergstrom, Gary C., Walker, Larry P., Gibson, Donna M. 2009. An optimized microplate assay system for quantitative evaluation of plant cell wall-degrading enzyme activity of fungal culture extracts. Biotechnology and Bioengineering, Vol. 102, pp. 1033-1044.

Noble, R., Fermor, T.R., Evered, C.E. and Atkey, P.T. 1997. Bench-scale preparation of mushroom substrates in controlled environments. Compost Science and Utilization, Vol. 5 (3), pp. 32-43.

## Claims

1. A bacterial composition comprising at least one strain of *Bacillus licheniformis* that is capable of (a) degrading complex carbohydrates selected from the group comprising: hemicellulose, cellulose, and combinations thereof, lignin, and protein; and (b) assimilating ammonia; wherein the at least one strain of *Bacillus licheniformis* comprises a gram positive, catalase positive and oxidase positive rod shaped strain that has a white coloured, rough surfaced colony morphology, and in which the at least one strain of *Bacillus licheniformis* comprises a strain selected from the group comprising
the *Bacillus licheniformis* strain deposited at NCIMB Ltd. with accession number NCIMB 42690 having a white colour, rough surface, irregular form, flat elevation, and curled margin colony morphology,
the *Bacillus licheniformis* strain deposited at NCIMB Ltd. with accession number NCIMB 42691 having a white colour, rough surface, filamentous form, raised elevation, and filamentous margin colony morphology,
and combinations thereof.

2. The bacterial composition according to claim 1 comprising the *Bacillus licheniformis* strain deposited at NCIMB Ltd. with accession number NCIMB 42690 and the *Bacillus licheniformis* strain deposited at NCIMB Ltd. with accession number NCIMB 42691.

3. A kit of parts for use in preparing augmented compost and/or farming, the kit of parts comprising the bacterial composition of Claims 1 or 2.

4. The kit of parts of Claim 3, further comprising instructions for use thereof.

5. A method for preparing augmented compost, the method comprising the steps of:
(i) providing the bacterial composition according to any preceding claim;
(ii) providing compost;
(iii) inoculating the compost with the bacterial composition of Claims 1 or 2.

6. The method of Claim 5, wherein the step of inoculating the compost with the bacterial composition is carried out under temperature-controlled conditions.

7. The method of Claims 5 or 6, wherein the step of inoculating the compost with the bacterial composition is carried out under aerobic conditions.

8. Augmented compost comprising the bacterial composition of Claims 1 or 2.

9. Use of augmented compost as a nutrient source for farming, the use comprising the steps of:
(i) providing the augmented compost prepared according to Claims 5, 6, or 7;
(ii) providing at least one fungus, mycelium, spawn, spore, plant, cutting, turf, and/or seed;
(iii) cultivating the at least one fungus, mycelium, spawn, spore, plant, cutting, turf, and/or seed in an environment comprising the augmented compost.

10. The use according to Claim 9, comprising the steps of:
(i) providing the augmented compost prepared according to Claims 5, 6, or 7;
(ii) providing at least one *Agaricus bisporus* mushroom, mycelium, spawn, and/or spore;
(iii) cultivating the at least one *Agaricus bisporus* mushroom, mycelium, spawn, and/or spore, in an environment comprising the augmented compost.

11. Use according to claim 9 or 10 as a nutrient source for fungus farming, preferably mushroom farming, more preferably *Agaricus bisporus* farming.

## Patentansprüche

1. Eine bakterielle Zusammensetzung, die mindestens einen Stamm von *Bacillus licheniformis* beinhaltet, der fähig ist zum (a) Abbauen von komplexen Kohlenhydraten, die aus der Gruppe ausgewählt sind, die Folgendes beinhaltet: Hemicellulose, Cellulose und Kombinationen davon, Lignin und Protein; und (b) Assimilieren von Ammoniak; wobei der mindestens eine Stamm von *Bacillus licheniformis* einen grampositiven, katalasepositiven und oxidasepositiven stäbchenförmigen Stamm beinhaltet, der eine weißfarbige Koloniemorphologie mit rauer Oberfläche aufweist und wobei der mindestens eine Stamm von *Bacillus licheniformis* einen Stamm beinhaltet, der aus der Gruppe ausgewählt ist, die Folgendes beinhaltet:
den bei NCIMB Ltd. mit der Zugangsnummer NCIMB 42690 hinterlegten Stamm von *Bacillus licheniformis,* der eine Koloniemorphologie mit weißer Farbe, rauer Oberfläche, unregelmäßiger Form, flachem Querschnitt und konzentrischem (curled) Rand aufweist, den bei NCIMB Ltd. mit der Zugangsnummer NCIMB 42691 hinterlegten Stamm von *Bacillus licheniformis,* der eine Koloniemorphologie mit weißer Farbe, rauer Oberfläche, fadenförmiger Form, erhabenem Querschnitt und fadenförmigem Rand aufweist, und Kombinationen davon.

2. Bakterielle Zusammensetzung gemäß Anspruch 1, die den bei NCIMB Ltd. mit der Zugangsnummer NCIMB 42690 hinterlegten Stamm von *Bacillus licheniformis* und den bei NCIMB Ltd. mit der Zugangsnummer NCIMB 42691 hinterlegten Stamm von *Bacillus licheniformis* beinhaltet.

3. Ein Kit aus Teilen zur Verwendung bei der Herstellung von angereichertem Kompost und/oder in der Landwirtschaft, wobei das Kit aus Teilen die bakterielle Zusammensetzung gemäß Anspruch 1 oder 2 beinhaltet.

4. Kit aus Teilen gemäß Anspruch 3, das ferner Anweisungen zu dessen Verwendung beinhaltet.

5. Ein Verfahren zum Herstellen von angereichertem Kompost, wobei das Verfahren die folgenden Schritte beinhaltet:
(i) Bereitstellen der bakteriellen Zusammensetzung gemäß einem der vorhergehenden Ansprüche;
(ii) Bereitstellen von Kompost;
(iii) Beimpfen des Komposts mit der bakteriellen Zusammensetzung gemäß Anspruch 1 oder 2.

6. Verfahren gemäß Anspruch 5, wobei der Schritt des Beimpfens des Komposts mit der bakteriellen Zusammensetzung unter temperaturgeregelten Bedingungen ausgeführt wird.

7. Verfahren gemäß Anspruch 5 oder 6, wobei der Schritt des Beimpfens des Komposts mit der bakteriellen Zusammensetzung unter aeroben Bedingungen ausgeführt wird.

8. Angereicherter Kompost, der die bakterielle Zusammensetzung gemäß Anspruch 1 oder 2 beinhaltet.

9. Verwendung von angereichertem Kompost als eine Nährstoffquelle für die Landwirtschaft, wobei die Verwendung die folgenden Schritte beinhaltet:
(i) Bereitstellen des gemäß den Ansprüchen 5, 6 oder 7 hergestellten angereicherten Komposts;
(ii) Bereitstellen von mindestens einem Pilz, einem Myzel, einer Pilzbrut, einer Spore, einer Pflanze, einem Steckling, einer Sode und/oder einem Samen;
(iii) Züchten des/der mindestens einen Pilzes, Myzels, Pilzbrut, Spore, Pflanze, Stecklings, Sode und/oder Samens in einer Umgebung, die den angereicherten Kompost beinhaltet.

10. Verwendung gemäß Anspruch 9, die die folgenden Schritte beinhaltet:
(i) Bereitstellen des gemäß den Ansprüchen 5, 6 oder 7 hergestellten angereicherten Komposts;
(ii) Bereitstellen mindestens eines Champignons, eines Myzels, einer Pilzbrut und/oder einer Spore von *Agaricus bisporus;*
(iii) Züchten des/der mindestens einen Champignons, Myzels, Pilzbrut und/oder Spore von *Agaricus bisporus* in einer Umgebung, die den angereicherten Kompost beinhaltet.

11. Verwendung gemäß Anspruch 9 oder 10 als eine Nährstoffquelle für den Pilzanbau, vorzugsweise den Anbau von Champignons, noch bevorzugter den Anbau von *Agaricus bisporus.*

## Revendications

1. Une composition bactérienne comprenant au moins une souche de *Bacillus licheniformis* qui est apte (a) à dégrader des glucides complexes sélectionnés dans le groupe comprenant : une hémicellulose, une cellulose, et des combinaisons de celles-ci, de la lignine, et une protéine ; et (b) à assimiler de l'ammoniac ; où l'au moins une souche de *Bacillus licheniformis* comprend une souche en forme de tige Gram positif, positive à la catalase et positive à l'oxydase qui a une morphologie de la colonie à surface rugueuse, de couleur blanche, et dans laquelle l'au moins une souche de *Bacillus licheniformis* comprend une souche sélectionnée dans le groupe comprenant la souche de *Bacillus licheniformis* déposée auprès de NCIMB Ltd. sous le numéro d'ordre NCIMB 42690 ayant une morphologie de la colonie à couleur blanche, surface rugueuse, forme irrégulière, élévation plate, et bordures recourbées,
la souche de *Bacillus licheniformis* déposée auprès de NCIMB Ltd. sous le numéro d'ordre NCIMB 42691 ayant une morphologie de la colonie à couleur blanche, surface rugueuse, forme filamenteuse, élévation élevée, et bordures filamenteuses,
et des combinaisons de celles-ci.

2. La composition bactérienne selon la revendication 1 comprenant la souche de *Bacillus licheniformis* déposée auprès de NCIMB Ltd. sous le numéro d'ordre NCIMB 42690 et la souche de *Bacillus licheniformis* déposée auprès de NCIMB Ltd. sous le numéro d'ordre NCIMB 42691.

3. Un kit de composants pour son utilisation dans la préparation de compost augmenté et/ou l'agriculture, le kit de composants comprenant la composition bactérienne des revendications 1 ou 2.

4. Le kit de composants de la revendication 3, comprenant en outre des instructions pour l'utilisation de celui-ci.

5. Un procédé pour la préparation de compost augmenté, le procédé comprenant les étapes :
(i) de fourniture de la composition bactérienne selon n'importe quelle revendication précédente ;
(ii) de fourniture de compost ;
(iii) d'inoculation du compost au moyen de la composition bactérienne des revendications 1 ou 2.

6. Le procédé de la revendication 5, où l'étape d'inoculation du compost au moyen de la composition bactérienne est réalisée dans des conditions de température contrôlée.

7. Le procédé des revendications 5 ou 6, où l'étape d'inoculation du compost au moyen de la composition bactérienne est réalisée dans des conditions aérobies.

8. Compost augmenté comprenant la composition bactérienne des revendications 1 ou 2.

9. Utilisation de compost augmenté comme source de nutriments pour l'agriculture, l'utilisation comprenant les étapes :
(i) de fourniture du compost augmenté préparé selon les revendications 5, 6, ou 7 ;
(ii) de fourniture d'au moins un(e) mycète, mycélium, blanc, spore, plante, bouture, motte, et/ou graine ;
(iii) de culture de l'au moins un(e) mycète, mycélium, blanc, spore, plante, bouture, motte, et/ou graine dans un environnement comprenant le compost augmenté.

10. L'utilisation selon la revendication 9, comprenant les étapes :
(i) de fourniture du compost augmenté préparé selon les revendications 5, 6, ou 7 ;
(ii) de fourniture d'au moins un champignon, mycélium, blanc, et/ou spore *d'Agaricus bisporus ;*
(iii) de culture de l'au moins un champignon, mycélium, blanc, et/ou spore *d'Agaricus bisporus,* dans un environnement comprenant le compost augmenté.

11. Utilisation selon la revendication 9 ou la revendication 10 comme source de nutriments pour la culture de mycètes, de préférence la culture de champignons, de préférence encore la culture *d'Agaricus bisporus.*
